(19) Europäisches Patentamt
European Patent Office
Office européen des brevets

(11) EP 4 269 491 A1

(12) EUROPEAN PATENT APPLICATION
published in accordance with Art. 153(4) EPC

(43) Date of publication:
**01.11.2023 Bulletin 2023/44**

(21) Application number: **21910935.2**

(22) Date of filing: **23.12.2021**

(51) International Patent Classification (IPC):
***C08K 5/3445** (2006.01)* ***C08L 21/00** (2006.01)*
***C08J 9/04** (2006.01)* ***C08J 9/06** (2006.01)*
***C08K 3/011** (2018.01)*

(52) Cooperative Patent Classification (CPC):
**C08J 9/04; C08J 9/06; C08K 3/011; C08K 5/3445; C08L 21/00**

(86) International application number:
**PCT/JP2021/047785**

(87) International publication number:
**WO 2022/138795 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020216785**
**30.04.2021 JP 2021077364**
**26.08.2021 JP 2021137854**

(71) Applicant: **Otsuka Chemical Co., Ltd.**
**Osaka-shi, Osaka 540-0021 (JP)**

(72) Inventor: **AOYAGI, Seiichi**
**Tokushima-shi, Tokushima 771-0193 (JP)**

(74) Representative: **Meissner Bolte Partnerschaft mbB**
**Widenmayerstrasse 47**
**80538 München (DE)**

(54) **RUBBER COMPOSITION FOR PRODUCING FOAM HAVING HIGH EXPANSION RATIO, FOAM HAVING HIGH EXPANSION RATIO, TIRE, ACOUSTIC MEMBER, SEALING MATERIAL, HOSE, BELT, WIRE COVERING, THERMAL INSULATION MATERIAL, EXPANSION RATIO IMPROVER FOR RUBBERS, METHOD FOR IMPROVING EXPANSION RATIO OF FOAM, AND METHOD FOR PRODUCING FOAM HAVING HIGH EXPANSION RATIO**

(57) Provided are a rubber composition for producing a foam having a high expansion ratio, a high-expansion-ratio foam, a tire, an acoustic member, a sealing material, a hose, a belt, a wire covering, a thermal insulation material, an expansion ratio improver for rubber, a method for improving the expansion ratio of a foam, and a method for producing a high-expansion-ratio foam. A rubber composition for producing a foam having a high expansion ratio, comprising the following components (a), (b), (c), and (d):
(a) a rubber component;
(b) a chemical foaming agent;
(c) a compound represented by the following formula (1) or a salt thereof; and
(d) at least one member selected from the group consisting of vulcanizing agents and crosslinking agents:

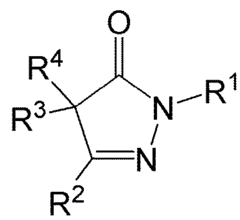
(1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

EP 4 269 491 A1

## Description

### Technical Field

The present invention relates to a rubber composition for producing a foam having a high expansion ratio, a high-expansion-ratio foam, a tire, an acoustic member, a sealing material, a hose, a belt, a wire covering, a thermal insulation material, an expansion ratio improver for rubber, a method for improving the expansion ratio of a foam, and a method for producing a high-expansion-ratio foam.

### Background Art

Rubber foams are used for various applications, such as tire members, acoustic members, sealing materials, hoses, and belts, because of their excellent lightweight and thermal insulation properties (PTL 1 and PTL 2).

In general, a rubber foam is produced by adding a chemical foaming agent to a rubber component, and applying heat thereto, followed by foaming with gas generated when the chemical foaming agent is decomposed. Although it depends on the application of the foam, the produced foam more preferably has a larger expansion ratio, in terms of production efficiency, the physical properties of the foam, etc.

As a method for producing a foam with a large expansion ratio, there is a method of adding a large amount of foaming agent to a rubber component. However, with this method, not enough heat is applied to the entire foaming agent, and the undecomposed foaming agent may remain in the foam. This residual foaming agent affects the physical properties (strength etc.) of the foam, which is undesirable. In addition, increasing the amount of foaming agent increases the cost.

Accordingly, there is a demand for methods for improving the expansion ratio of foams by means other than increasing the use of foaming agents.

### Citation List

### Patent Literature

PTL 1: JP2020-122160A
PTL 2: JP2002-165294A

### Summary of Invention

### Technical Problem

In view of the above circumstances, an object of the present invention is to provide a rubber composition for producing a foam having a high expansion ratio, a high-expansion-ratio foam, a tire, an acoustic member, a sealing material, a hose, a belt, a wire covering, a thermal insulation material, an expansion ratio improver for rubber, a method for improving the expansion ratio of a foam, and a method for producing a high-expansion-ratio foam.

### Solution to Problem

As a result of extensive research to achieve the above object, the present inventor found that a rubber composition for producing a foam having a high expansion ratio can be obtained by adding a pyrazolone-based compound to a rubber component containing a chemical foaming agent. Upon further research based on this finding, the present inventor has completed the present invention.

Specifically, the present invention provides a rubber composition for producing a foam having a high expansion ratio, a high-expansion-ratio foam, a tire, an acoustic member, a sealing material, a hose, a belt, a wire covering, a thermal insulation material, an expansion ratio improver for rubber, a method for improving the expansion ratio of a foam, and a method for producing a high-expansion-ratio foam, described below.

Item 1.

A rubber composition for producing a foam having a high expansion ratio, comprising the following components (a), (b), (c), and (d):

(a) a rubber component;
(b) a chemical foaming agent;
(c) a compound represented by the following formula (1) or a salt thereof; and
(d) at least one member selected from the group consisting of vulcanizing agents and crosslinking agents:

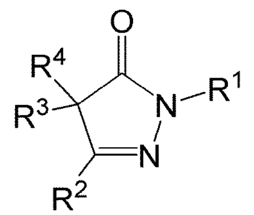

(1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

Item 2.

The composition according to Item 1, wherein the rubber component is at least one diene rubber selected from the group consisting of natural rubber, styrene-butadiene copolymer rubber (SBR), butadiene rubber (BR), isoprene rubber (IR), styrene-isoprene-butadiene rubber (SIBR), nitrile rubber (NBR), chloroprene rubber (CR), a styrene-isoprene-styrene triblock copolymer (SIS), and a styrene-butadiene-styrene triblock copolymer (SBS).

Item 3.

The composition according to Item 1, wherein the rubber component is at least one non-diene rubber selected from the group consisting of butyl rubber, ethylene-propylene rubber (EPM), ethylene-propylene-diene terpolymer rubber (EPDM), urethane rubber (U), a propylene hexafluoride-vinylidene fluoride copolymer (FKM), a tetrafluoroethylene-propylene copolymer (FEPM), a tetrafluoroethylene-perfluorovinyl ether copolymer (FFKM), methyl silicone rubber (MQ), vinyl methyl silicone rubber (VMQ), phenyl methyl silicone rubber (PMQ), acrylic rubber (ACM), polysulfide rubber (T), and epichlorohydrin rubber (CO, ECO).

Item 4.

The composition according to any one of Items 1 to 3, wherein $R^1$, $R^3$, and $R^4$ are hydrogen atoms, and $R^2$ is a methyl group.

Item 5.

The composition according to any one of Items 1 to 4, further comprising component (e): a foaming aid.

Item 6.

A high-expansion-ratio foam foamed from the composition according to any one of Items 1 to 5.

Item 7.

A tire, an acoustic member, a sealing material, a hose, a belt, a wire covering, or a thermal insulation material, all of which are produced by using the composition according to any one of Items 1 to 5 or the foam according to Item 6.

Item 8.

An expansion ratio improver for rubber, comprising a compound represented by the following formula (1) or a salt thereof:

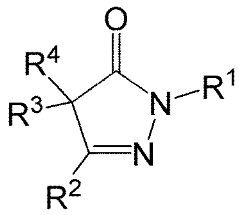

(1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

Item 9.

A method for improving the expansion ratio of a foam, comprising mixing a rubber component, a chemical foaming agent, and a compound represented by the following formula (1) or a salt thereof:

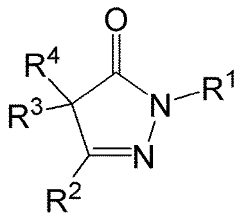

(1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

Item 10.

A method for producing a high-expansion-ratio foam, comprising:

step (A) of mixing a raw material component containing components (a) and (c); and
step (B) of mixing a mixture obtained in step (A) with components (b) and (d).

Item 11.

The method for producing a high-expansion-ratio foam according to Item 10, wherein the foam has an expansion ratio index of 103 or more.

Advantageous Effects of Invention

The rubber composition for producing a foam having a high expansion ratio according to the present invention as described above has an excellent expansion ratio.

Description of Embodiments

The present invention is described in detail below.

1. Rubber Composition for Producing Foam Having High Expansion Ratio

The rubber composition for producing a foam having a high expansion ratio of the present invention comprises the following components (a), (b), (c), and (d):

(a) a rubber component;
(b) a chemical foaming agent;
(c) a compound represented by the following formula (1) or a salt thereof; and

(d) at least one member selected from the group consisting of vulcanizing agents and crosslinking agents:

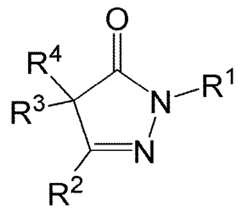

(1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

A rubber composition for producing a foam having a high expansion ratio can be obtained by incorporating components (a), (b), (c), and (d).

The rubber composition for producing a foam having a high expansion ratio of the present invention may be simply a mixture of components (a), (b), (c), and (d), or after components (a), (b), (c), and (d) are mixed, component (b) may be foamed. That is, components (a), (b), (c), and (d) may be mixed in any order.

1.1. Component (a): Rubber Component

Component (a) is a rubber component. The rubber component is not particularly limited, and examples include diene rubbers, non-diene rubbers, and mixtures of diene rubbers and non-diene rubbers.

Examples of diene rubbers include natural rubber, styrene-butadiene copolymer rubber (SBR), butadiene rubber (BR), isoprene rubber (IR), styrene-isoprene-butadiene rubber (SIBR), nitrile rubber (NBR), chloroprene rubber (CR), a styrene-isoprene-styrene triblock copolymer (SIS), a styrene-butadiene-styrene triblock copolymer (SBS), and modified diene rubbers thereof. Preferred among these are natural rubber, butadiene rubber, and styrene-butadiene copolymer rubber.

Examples of natural rubbers include natural rubber latex, technically specified rubber (TSR), smoked sheet (RSS), gutta-percha, natural rubber from *Eucommia ulmoides,* natural rubber from guayule, natural rubber from Russian dandelion, and the like. Further, it is also preferable to use modified natural rubbers obtained by modifying these natural rubbers, such as epoxidized natural rubber, methacrylic acid-modified natural rubber, halogen-modified natural rubber, deproteinized natural rubber, maleic acid-modified natural rubber, sulfonic acid-modified natural rubber, and styrene-modified natural rubber.

Modified diene rubbers include diene rubbers modified by main-chain modification, single-end modification, both-end modification, or the like. Modified functional groups of modified diene rubbers include epoxide, amino, alkoxysilyl, hydroxyl, and other various functional groups. Modified diene rubbers may contain one or two or more of these functional groups.

The method for producing diene rubber is not particularly limited, and examples include emulsion polymerization, solution polymerization, radical polymerization, anionic polymerization, cationic polymerization, and the like. There is no particular restriction on the glass transition point of synthetic diene rubber.

Examples of non-diene rubbers include butyl rubber, ethylene-propylene rubber (EPM), ethylene-propylene-diene terpolymer rubber (EPDM), urethane rubber (U), a propylene hexafluoride-vinylidene fluoride copolymer (FKM), a tetrafluoroethylene-propylene copolymer (FEPM), a tetrafluoroethylene-perfluorovinyl ether copolymer (FFKM), methyl silicone rubber (MQ), vinyl methyl silicone rubber (VMQ), phenyl methyl silicone rubber (PMQ), acrylic rubber (ACM), polysulfide rubber (T), epichlorohydrin rubber (CO, ECO), and modified non-diene rubbers thereof. Preferred among these are butyl rubber and ethylene-propylene-diene terpolymer rubber (EPDM).

Modified non-diene rubbers include non-diene rubbers modified by main-chain modification, single-end modification, both-end modification, or the like. Modified functional groups of modified non-diene rubbers include epoxide, amino, alkoxysilyl, hydroxyl, and other various functional groups. Modified synthetic non-diene rubbers may contain one or two or more of these functional groups.

The method for producing non-diene rubber is not particularly limited, and examples include emulsion polymerization, solution polymerization, radical polymerization, anionic polymerization, cationic polymerization, and the like. There is no particular restriction on the glass transition point of synthetic non-diene rubber.

The ratio of cis/trans/vinyl at the double bond of natural rubber and diene rubber is not particularly limited, and any ratio can be suitably used. Further, the number average molecular weight and molecular weight distribution of diene rubber are also not particularly limited. The number average molecular weight is preferably 500 to 3000000, and the

molecular weight distribution is preferably 1.5 to 15. As the non-diene rubber, known rubbers can be widely used.

The rubber components can be used singly or as a mixture (blend) of two or more. Among these, the rubber component is preferably a mixture of natural rubber and butadiene rubber.

The content of component (a) in 100 mass% in total of components (a), (b), (c), and (d) in the rubber composition is preferably 1 to 99 mass%, and more preferably 5 to 98 mass%. Because 1 mass% or more of component (a) is contained, elasticity can be imparted to the rubber composition. On the other hand, the content of component (a) in the rubber composition being set to 99 mass% or less can reduce the cost of the rubber composition and consequently improve economic efficiency.

1.2. Component (b): Chemical Foaming Agent

The chemical foaming agent is not particularly limited, and known chemical foaming agents can be widely used. Examples include organic chemical foaming agents, such as azodicarbonamide, N,N'-dinitrosopentanemethylenetetramine, p,p'-oxybisbenzenesulfonyl hydrazide, p-toluenesulfonyl hydrazide, p-toluenesulfonyl semicarbazide, diazoaminobenzene, hydrazodicarbonamide, barium azodicarboxylate, azobisisobutyronitrile, monosodium citrate, and other organic acids and metal salts thereof; and inorganic chemical foaming agents, such as sodium bicarbonate, ammonium hydrogen carbonate, sodium carbonate, ammonium carbonate, aluminum acetate, ammonium nitrite, and sodium borohydride.

These chemical foaming agents can be used singly or as a mixture (blend) of two or more.

Preferred among the above chemical foaming agents is azodicarbonamide, p,p'-oxybisbenzenesulfonyl hydrazide, or sodium bicarbonate.

The surface of these foaming agents may be chemically treated. Such foaming agents are excellent in terms of prevention of their solidification, dispersibility, dust control, workability, storage stability, etc. Further, compositions and foams obtained by using such foaming agents are excellent in terms of improvement of mechanical properties, miniaturization of cells, etc.

The median diameter of azodicarbonamide is preferably 0.1 to 1,000 pm, more preferably 1 to 100 pm, and particularly preferably 1 to 50 um. The median diameter of azodicarbonamide being set to 0.1 to 1,000 um can improve dispersibility and cell uniformity.

The amount of component (b) blended is preferably 0.1 to 100 parts by mass, more preferably 0.1 to 80 parts by mass, even more preferably 0.5 to 60 parts by mass, and particularly preferably 1 to 50 parts by mass, based on 100 parts by mass of component (a) in the rubber composition.

The content of component (b) in 100 mass% in total of components (a), (b), (c), and (d) in the rubber composition is preferably 0.01 to 95 mass%, more preferably 0.05 to 90 mass%, even more preferably 0.1 to 50 mass%, and particularly preferably 0.5 to 30 mass%. Because 0.01 mass% or more of component (b) is contained, the expansion ratio of component (a) can be improved. On the other hand, the content of component (b) in the rubber composition being set to 95 mass% or less can reduce the cost of the rubber composition and improve economic efficiency.

1.3. Component (c): Compound Represented by Formula (1) or Salt Thereof

Component (c) is a compound represented by the following formula (1) or a salt thereof (hereinafter the compound and a salt thereof are also collectively referred to simply as "compound (1) ") .

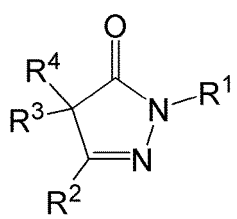

(1)

In formula (1), $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally has one or more substituents.

The "alkyl group" in compound (1) is not particularly limited, and examples include linear, branched, or cyclic alkyl groups. Specific examples include

$C_{1-4}$ linear or branched alkyl groups, such as methyl, ethyl, n-propyl, isopropyl, n-butyl, isobutyl, s-butyl, and t-butyl;

$C_{5-18}$ linear or branched alkyl groups, such as 1-ethylpropyl, n-pentyl, isopentyl, neopentyl, n-hexyl, isohexyl,

3-methylpentyl, n-heptyl, n-octyl, n-nonyl, n-decyl, n-undecyl, n-dodecyl, 5-propylnonyl, n-tridecyl, n-tetradecyl, n-pentadecyl, hexadecyl, heptadecyl, and octadecyl;

$C_{3-8}$ cyclic alkyl groups, such as cyclopropyl, cyclobutyl, cyclopentyl, cyclohexyl, cycloheptyl, and cyclooctyl; and the like.

The "aralkyl group" in compound (1) is not particularly limited, and examples include benzyl, phenethyl, trityl, 1-naphthylmethyl, 2-(1-naphthyl)ethyl, and 2-(2-naphthyl)ethyl groups.

The "aryl group" in compound (1) is not particularly limited, and examples include phenyl, biphenyl, naphthyl, dihydroindenyl, and 9H-fluorenyl groups.

These alkyl, aralkyl, and aryl groups optionally have one or more substituents at any replaceable position. Such substituents are not particularly limited, and examples include halogen, amino, aminoalkyl, alkoxycarbonyl, acyl, acyloxy, amide, carboxyl, carboxyalkyl, formyl, nitrile, nitro, alkyl, hydroxyalkyl, hydroxyl, alkoxy, aryl, aryloxy, thiol, alkylthio, and arylthio groups. The number of substituents is preferably 1 to 5, and more preferably 1 to 3.

Examples of the "halogen atom" in compound (1) include fluorine, chlorine, bromine, iodine, and astatine atoms; and preferably fluorine, chlorine, bromine, and iodine atoms.

Examples of the "amino group" in compound (1) include not only an amino group represented by $-NH_2$, but also linear or branched monoalkyl amino groups having about 1 to 6 carbon atoms, such as methylamino, ethylamino, n-propylamino, isopropylamino, n-butylamino, isobutylamino, s-butylamino, t-butylamino, 1-ethylpropylamino, n-pentylamino, neopentylamino, n-hexylamino, isohexylamino, and 3-methylpentylamino groups; and substituted amino groups, such as dialkyl amino groups having two linear or branched alkyl groups having about 1 or 2 carbon atoms, such as dimethylamino, ethylmethylamino, and diethylamino groups.

The "aminoalkyl group" in compound (1) is not particularly limited, and examples include aminoalkyl groups, monoalkyl-substituted aminoalkyl groups, or dialkyl-substituted aminoalkyl groups, which have about 1 to 7 carbon atoms, such as aminomethyl, methylamino methyl, ethylamino methyl, dimethylamino methyl, ethyl methylamino methyl, diethylamino methyl, 2-aminoethyl, 2-(methylamino)ethyl, 2-(ethylamino)ethyl, 2-(dimethylamino)ethyl, 2-(ethylmethylamino)ethyl, 2-(diethylamino)ethyl, 3-aminopropyl, 3-(methylamino)propyl, 3-(ethylamino)propyl, 3-(dimethylamino)propyl, 3-(ethylmethylamino)propyl, and 3-(diethylamino)propyl groups; and the like.

The "alkoxycarbonyl group" in compound (1) is not particularly limited, and examples include $C_{1-4}$ linear or branched alkoxycarbonyl groups, such as methoxycarbonyl and ethoxycarbonyl groups.

The "acyl group" in compound (1) is not particularly limited, and examples include $C_{1-4}$ linear or branched alkylcarbonyl groups, such as acetyl, propionyl, and pivaloyl groups.

The "acyloxy group" in compound (1) is not particularly limited, and examples include $C_{1-4}$ linear or branched acyloxy groups, such as acetyloxy, propionyloxy, and n-butyryloxy groups.

The "amide group" in compound (1) is not particularly limited, and examples include carboxylic acid amide groups, such as acetamide and benzamide groups; thioamide groups, such as thioacetamide and thiobenzamide groups; N-substituted amide groups, such as N-methylacetamide and N-benzylacetamide groups; and the like.

The "carboxyalkyl group" in compound (1) is not particularly limited, and examples include carboxyalkyl groups, such as carboxymethyl, carboxyethyl, carboxy-n-propyl, carboxy-n-butyl, carboxy-n-pentyl, and carboxy-n-hexyl groups.

The "hydroxyalkyl group" in compound (1) is not particularly limited, and examples include hydroxyalkyl groups, such as hydroxymethyl, hydroxyethyl, hydroxy-n-propyl, and hydroxy-n-butyl groups.

The "alkoxy group" in compound (1) is not particularly limited, and examples include linear, branched, or cyclic alkoxy groups. Specific examples include linear or branched alkoxy groups, such as methoxy, ethoxy, n-propoxy, isopropoxy, n-butoxy, t-butoxy, n-pentyloxy, neopentyloxy, and n-hexyloxy groups; cyclic alkoxy groups, such as cyclopropyloxy, cyclobutyloxy, cyclopentyloxy, cyclohexyloxy, cycloheptyloxy, and cyclooctyloxy groups; and the like.

The "aryloxy group" in compound (1) is not particularly limited, and examples include phenoxy, biphenyloxy, and naphthoxy groups.

The "alkylthio group" in compound (1) is not particularly limited, and examples include methylthio, ethylthio, and n-propylthio groups.

The "arylthio group" in compound (1) is not particularly limited, and examples include phenylthio, naphthylthio, and biphenylthio groups.

Preferred among the compounds represented by formula (1) is a compound wherein $R^1$ is a hydrogen atom.

Preferred among the compounds represented by formula (1) is a compound wherein $R^2$ is a hydrogen atom, a $C_{1-4}$ linear or branched alkyl group, an aralkyl group, or an aryl group; more preferred is a compound wherein $R^2$ is a hydrogen atom or a $C_{1-4}$ linear or branched alkyl group; and even more preferred is a compound wherein $R^2$ is a methyl group.

Preferred among the compounds represented by formula (1) is a compound wherein at least one of $R^3$ and $R^4$ is a hydrogen atom, and more preferred is a compound wherein $R^3$ and $R^4$ are both hydrogen atoms.

Preferred among the compounds represented by formula (1) is a compound wherein $R^1$ is a hydrogen atom, $R^2$ is a hydrogen atom, a $C_{1-4}$ linear or branched alkyl group, an aralkyl group, or an aryl group, and $R^3$ and $R^4$ are both

hydrogen atoms; more preferred is a compound wherein $R^1$ is a hydrogen atom, $R^2$ is a hydrogen atom or a $C_{1-4}$ linear or branched alkyl group, and $R^3$ and $R^4$ are both hydrogen atoms; and particularly preferred is a compound wherein $R^1$ is a hydrogen atom, $R^2$ is a methyl group, and $R^3$ and $R^4$ are both hydrogen atoms.

Examples of the compound represented by formula (1) include 5-pyrazolone, 3-methyl-5-pyrazolone, 3-(naphthalen-2-yl)-1H-pyrazol-5(4H)-one, 3-phenyl-1H-pyrazol-5(4H)-one, 3-propyl-1H-pyrazol-5(4H)-one, 3-undecyl-1H-pyrazol-5(4H)-one, 4-(2-hydroxyethyl)-3-methyl-1H-pyrazol-5(4H)-one, 4-benzyl-3-methyl-1H-pyrazol-5(4H)-one, 5-methyl-2-(4-nitrophenyl)-1H-pyrazol-3(2H)-one, and the like.

More preferred among these as the compound represented by formula (1) are 5-pyrazolone, 3-methyl-5-pyrazolone, 3-(naphthalen-2-yl)-1H-pyrazol-5(4H)-one, 3-phenyl-1H-pyrazol-5(4H)-one, and 3-propyl-1H-pyrazol-5(4H)-one; and particularly preferred is 3-methyl-5-pyrazolone.

As component (c) in the rubber composition for producing a foam having a high expansion ratio of the present invention, the above compounds may be contained singly or in combination of two or more.

Some of compounds (1) generate tautomers. Chemical equilibrium of tautomers can be achieved if tautomerization is possible (e.g., in a solution). Compounds (1) can be present as, for example, tautomers represented by formulas (2) to (7).

The compound of formula (1) wherein $R^1$ and $R^3$ are hydrogen atoms (compound (1)-A) has tautomers represented by the following formulas (2) to (4):

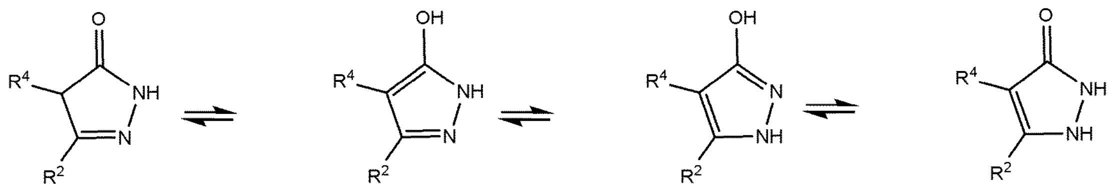

(1)-A (2) (3) (4)

wherein $R^2$ and $R^4$ are as defined above.

The compound of formula (1) wherein $R^3$ is a hydrogen atom (compound (1)-B) has tautomers represented by the following formulas (5) and (6):

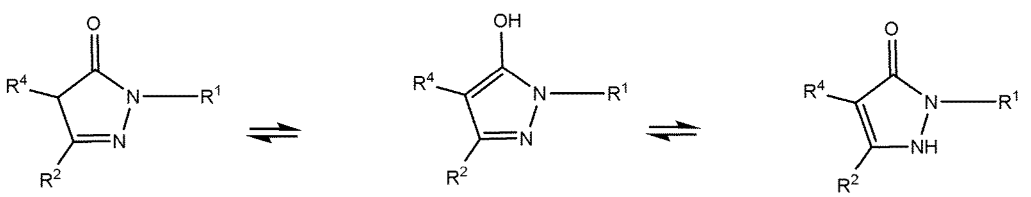

(1)-B (5) (6)

wherein $R^1$, $R^2$, and $R^4$ are as defined above.

The compound of formula (1) wherein $R^1$ is a hydrogen atom (compound (1)-C) has a tautomer represented by the following formula (7):

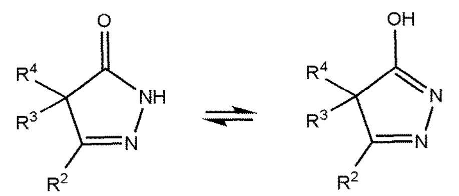

(1)-C (7)

wherein $R^2$, $R^3$, and $R^4$ are as defined above.

The tautomers represented by formulas (2) to (7) and compound (1) reach an equilibrium state in which both

isomers coexist. Therefore, unless otherwise specified, in the present specification, all the tautomers of compound (1) fall within the scope of the present invention.

The salts of the compound represented by formula (1) are not particularly limited and include various types of salts. Examples of such salts include inorganic acid salts, such as hydrochloride, sulfate, and nitrate; organic acid salts, such as acetate and methanesulfonate; alkali metal salts, such as sodium salt and potassium salt; alkaline earth metal salts, such as magnesium salt and calcium salt; ammonium salts, such as dimethylammonium and triethylammonium; and the like.

As component (c) in the rubber composition for producing a foam having a high expansion ratio of the present invention, a mixture containing compound (1) at any ratio may also be contained.

The amount of component (c) blended is preferably 0.01 to 50 parts by mass, more preferably 0.05 to 30 parts by mass, even more preferably 0.1 to 20 parts by mass, and particularly preferably 0.3 to 10 parts by mass, based on 100 parts by mass of component (a) in the rubber composition for producing a foam having a high expansion ratio.

The content of component (c) in 100 mass% in total of components (a), (b), (c), and (d) in the rubber composition is preferably 0.01 to 50 mass%, more preferably 0.05 to 30 mass%, even more preferably 0.1 to 20 mass%, and particularly preferably 0.2 to 6 mass%. Because 0.01 mass% or more of component (c) is contained, the expansion ratio of component (a) can be improved. On the other hand, the content of component (c) in the rubber composition being set to 50 mass% or less can reduce the cost of the rubber composition and improve economic efficiency.

Regarding the blending ratio of components (b) and (c), the ratio of component (c) is preferably 0.1 to 80 parts by mass, more preferably 1 to 75 parts by mass, even more preferably 3 to 70 parts by mass, and particularly preferably 5 to 65 parts by mass, based on the total mass of components (b) and (c), which is taken as 100 parts by mass.

1.4. Component (d): At Least One Member Selected from Group Consisting of Vulcanizing Agents and Crosslinking Agents

Component (d) is at least one member selected from the group consisting of vulcanizing agents and crosslinking agents. Vulcanizing agents and crosslinking agents can be used singly, or vulcanizing agents and crosslinking agents can be used together.

The vulcanizing agent is preferably sulfur, tetramethylthiuram disulfide, tetraethylthiuram disulfide, tetrabutylthiuram disulfide, tetrakis-(2-ethylhexyl)thiuram disulfide, dipentamethylenethiuram tetrasulfide, or the like. Preferred among these is sulfur. The vulcanizing agents may be contained singly or as a mixture of two or more.

The crosslinking agent is preferably dicumyl peroxide, benzoyl peroxide, dihexyl peroxide, di-t-butylperoxy diisopropylbenzene, 2,5-dimethyl-2,5-di-(t-butylperoxy)hexane, 2,5-dimethyl-2,5-di-(t-butylperoxy)hexyne-3, p-benzoquinone dioxime, lead oxide, zinc oxide, mercaptobenzothiazole, 2,2'-dibenzothiazolyl disulfide, or the like. Preferred among these is dicumyl peroxide. The crosslinking agents may be contained singly or as a mixture of two or more.

The amount of component (d) blended is preferably 0.01 to 10 parts by mass, more preferably 0.05 to 8 parts by mass, even more preferably 0.1 to 7 parts by mass, and particularly preferably 0.3 to 5 parts by mass, based on 100 parts by mass of component (a) in the rubber composition for producing a foam having a high expansion ratio.

The content of component (d) in 100 mass% in total of components (a), (b), (c), and (d) in the rubber composition is preferably 0.01 to 10 mass%, more preferably 0.05 to 8 mass%, even more preferably 0.1 to 5 mass%, and particularly preferably 0.3 to 3 mass%. Because 0.01 mass% or more of component (d) is contained, crosslinking is possible. On the other hand, the content of component (d) in the rubber composition being set to 10 mass% or less can reduce the cost of the composition and lower the odor.

1.5. Component (e): Foaming Aid

The rubber composition for producing a foam having a high expansion ratio of the present invention preferably further contains a foaming aid as component (e). The foaming aid is not particularly limited, and conventionally used foaming aids can be used. Examples include urea compounds, such as urea, mixtures of urea, fatty acids, and fatty acid metal salts, thiourea, tetramethylurea, dimethylthiourea, semicarbazide, and carbohydrazide; zinc compounds, such as zinc oxide, zinc stearate, zinc benzenesulfinate, zinc toluenesulfonate, zinc trifluoromethanesulfonate, and zinc carbonate; lead compounds, such as lead dioxide and tribasic lead; and the like.

The above foaming aids can be used singly or as a mixture (blend) of two or more.

The amount of component (e) blended is preferably 0.05 to 100 parts by mass, more preferably 0.1 to 80 parts by mass, even more preferably 0.5 to 60 parts by mass, and particularly preferably 1 to 30 parts by mass, based on 100 parts by mass of component (a) in the rubber composition.

The urea compound is preferably used in combination with component (b), such as azodicarbonamide, N,N'-dinitrosopentanemethylenetetramine, p,p'-oxybisbenzenesulfonyl hydrazide, p-toluenesulfonyl hydrazide, p-toluenesulfonyl semicarbazide, or hydrazodicarbonamide, and more preferably used in combination with azodicarbonamide.

The addition of the above urea compound to the rubber composition of the present invention can accelerate the decomposition of the foaming agent.

Further, the amount of component (b) is preferably 1 to 1000 parts by mass based on 100 parts by mass of the urea compound.

The zinc compound is preferably used in combination with component (b), such as azodicarbonamide, N,N'-dinitrosopentanemethylenetetramine, p,p'-oxybisbenzenesulfonyl hydrazide, p-toluenesulfonyl hydrazide, p-toluenesulfonyl semicarbazide, or hydrazodicarbonamide.

Further, the amount of component (b) is preferably 1 to 1000 parts by mass based on 100 parts by mass of the zinc compound.

The lead compound is preferably used in combination with component (b), such as azodicarbonamide, N,N'-dinitrosopentanemethylenetetramine, p,p'-oxybisbenzenesulfonyl hydrazide, p-toluenesulfonyl hydrazide, p-toluenesulfonyl semicarbazide, or hydrazodicarbonamide.

Further, the amount of component (b) is preferably 1 to 1000 parts by mass based on 100 parts by mass of the lead compound.

The content of component (e) in 100 mass% in total of components (a), (b), (c), (d), and (e) in the rubber composition is preferably 0.01 to 50 mass%, more preferably 0.05 to 40 mass%, and even more preferably 0.1 to 20 mass%. Because 0.01 mass% or more of component (e) is contained, the expansion ratio of component (a) can be improved. On the other hand, the content of component (e) in the rubber composition being set to 50 mass% or less can reduce the cost of the rubber composition and improve economic efficiency.

1.6. Other Ingredients

In addition to components (a), (b), (c), (d), and (e) described above, the rubber composition for producing a foam having a high expansion ratio of the present invention may contain ingredients generally used in the rubber industry. Examples include carbon black, inorganic fillers, antiaging agents, antiozonants, softeners, processing aids, waxes, resins, oils, $C_{8-30}$ fatty acids such as stearic acid, zinc oxide (ZnO), vulcanization accelerators, vulcanization retarders, and the like, which can be appropriately selected and blended within a range that does not impair the object of the present invention. Commercially available products can be suitably used as these ingredients.

Carbon black is generally used to improve rubber toughness. In the present specification, inorganic fillers do not include carbon black.

Examples of carbon black include, but are not particularly limited to, commercially available carbon black, carbon-silica dual phase filler. Because the rubber component contains carbon black, the effect of reducing the electrical resistance of the rubber, the effect of suppressing electrification, and the effect of improving the strength of the rubber can be appreciated.

Specific example of carbon black include high-, middle-, or low-structure SAF, ISAF, IISAF, N110, N134, N220, N234, N330, N339, N375, N550, HAF, FEF, GPF, and SRF grade carbon black. Preferred carbon black among these is SAF, ISAF, IISAF, N134, N234, N330, N339, N375, HAF, or FEF grade carbon black.

The DBP absorption of carbon black is not particularly limited, and is preferably 60 to 200 $cm^3$/100 g, more preferably 70 to 180 $cm^3$/100 g, and particularly preferably 80 to 160 $cm^3$/100 g.

Further, the nitrogen adsorption specific surface area (N2SA, measured according to JISK6217-2: 2001) of carbon black is preferably 30 to 200 $m^2$/g, more preferably 40 to 180 $m^2$/g, and particularly preferably 50 to 160 $m^2$/g.

The inorganic filler is not particularly limited as long as it is an inorganic compound generally used in the rubber industry. Examples of usable inorganic compounds include silica; alumina ($Al_2O_3$), such as $\gamma$-alumina and $\alpha$-alumina; alumina monohydrate ($Al_2O_3 \cdot H_2O$), such as boehmite and diaspore; aluminum hydroxide [$Al(OH)_3$], such as gibbsite and bayerite; aluminum carbonate [$Al_2(CO_3)_3$], magnesium hydroxide [$Mg(OH)_2$], magnesium oxide (MgO), magnesium carbonate ($MgCO_3$), talc ($3MgO \cdot 4SiO_2 \cdot H_2O$), attapulgite ($5MgO \cdot 8SiO_2 \cdot 9H_2O$), titanium white ($TiO_2$), titanium black ($TiO_{2n-1}$), calcium oxide (CaO), calcium hydroxide [$Ca(OH)_2$], magnesium aluminum oxide ($MgO \cdot Al_2O_3$), clay ($Al_2O_3 \cdot 2SiO_2$), kaolin ($Al_2O_3 \cdot 2SiO_2 \cdot 2H_2O$), pyrophyllite ($Al_2O_3 \cdot 4SiO_2 \cdot H_2O$), bentonite ($Al_2O_3 \cdot 4SiO_2 \cdot 2H_2O$), aluminum silicate ($Al_2SiO_5$, $Al_4 \cdot 3SiO_4 \cdot SH_2O$, etc.), magnesium silicate ($Mg_2SiO_4$, $MgSiO_3$, etc.), calcium silicate ($Ca_2 \cdot SiO_4$, etc.), aluminum calcium silicate ($Al_2O_3 \cdot CaO \cdot 2SiO_2$, etc.), magnesium calcium silicate ($CaMgSiO_4$), calcium carbonate ($CaCO_3$), zirconium oxide ($ZrO_2$), zirconium hydroxide [$ZrO(OH)_2 \cdot nH_2O$], zirconium carbonate [$Zr(CO_3)_2$], zinc acrylate, zinc methacrylate, crystalline aluminosilicates that contain hydrogen, an alkali metal, or an alkaline earth metal and that correct charge like various zeolites, and the like. These inorganic fillers may be organically treated on their surface in order to improve the compatibility with the rubber component.

The inorganic filler is preferably silica in terms of imparting rubber strength, and more preferably silica alone or a combination of silica and one or more inorganic compounds generally used in the rubber industry. When silica and an inorganic compound other than silica are used in combination as the inorganic filler, the total amount of all components of the inorganic filler may be adjusted appropriately within the above range. Silica is preferably added because it can

impart rubber strength.

Any commercially available silica can be used. Among them, preferred silica is wet silica, dry silica, or colloidal silica, and more preferably wet silica. Such silica may be organically treated on the surface in order to improve the compatibility with the rubber component.

The BET specific surface area of silica is not particularly limited, and is in the range of 40 to 350 $m^2/g$, for example. Silica with a BET specific surface area in this range has the advantage of achieving both rubber toughness and dispersibility in the rubber component. The BET specific surface area is measured according to ISO 5794/1.

From this point of view, the silica is preferably silica with a BET specific surface area in the range of 80 to 300 $m^2/g$, more preferably silica with a BET specific surface area of 100 to 270 $m^2/g$, and particularly preferably silica with a BET specific surface area of 110 to 270 $m^2/g$.

Commercial products of silica include trade names HD165MP (BET specific surface area = 165 $m^2/g$), HD115MP (BET specific surface area = 115 $m^2/g$), HD200MP (BET specific surface area = 200 $m^2/g$), and HD250MP (BET specific surface area = 250 $m^2/g$), produced by Quechen Silicon Chemical Co., Ltd.; trade names Nipsil AQ (BET specific surface area = 205 $m^2/g$) and Nipsil KQ (BET specific surface area = 240 $m^2/g$), produced by Tosoh Silica Corporation; trade name Ultrasil VN3 (BET specific surface area = 175 $m^2/g$) produced by Degussa; and the like.

When the rubber composition for producing a foam having a high expansion ratio of the present invention contains carbon black, the amount of carbon black blended is not particularly limited, and is generally, for example, 1 to 200 parts by mass, preferably 2 to 150 parts by mass, and more preferably 3 to 120 parts by mass, based on 100 parts by mass of component (a) in the rubber composition.

When the rubber composition for producing a foam having a high expansion ratio of the present invention contains an inorganic filler, the amount of the inorganic filler blended is not particularly limited, and is generally, for example, 1 to 200 parts by mass, preferably 2 to 150 parts by mass, and more preferably 3 to 120 parts by mass, based on 100 parts by mass of component (a).

When the rubber composition for producing a foam having a high expansion ratio of the present invention contains both carbon black and an inorganic filler, the total amount of both components may be suitably adjusted within the above range.

Further, in the rubber composition in which the carbon black and/or inorganic filler mentioned above are blended, a silane coupling agent, a titanate coupling agent, an aluminate coupling agent, or a zirconate coupling agent may be blended for the purpose of increasing the toughness of the rubber composition or increasing both the tear strength and wear resistance of the rubber composition, by carbon black and/or silica.

Silane coupling agents that can be used in combination with the carbon black and/or inorganic filler are not particularly limited, and commercial products can be suitably used. Examples of such silane coupling agents include sulfide-based, polysulfide-based, thioester-based, thiol-based, olefin-based, epoxy-based, amino-based, and alkyl-based silane coupling agents.

Examples of sulfide-based silane coupling agents include bis(3-triethoxysilylpropyl)tetrasulfide, bis(3-trimethoxysilylpropyl)tetrasulfide, bis(3-methyldimethoxysilylpropyl)tetrasulfide, bis(2-triethoxysilylethyl)tetrasulfide, bis(3-triethoxysilylpropyl)disulfide, bis(3-trimethoxysilylpropyl)disulfide, bis(3-methyldimethoxysilylpropyl)disulfide, bis(2-triethoxysilylethyl)disulfide, bis(3-triethoxysilylpropyl)trisulfide, bis(3-trimethoxysilylpropyl)trisulfide, bis(3-methyldimethoxysilylpropyl)trisulfide, bis(2-triethoxysilylethyl)trisulfide, bis(3-monoethoxydimethylsilylpropyl)tetrasulfide, bis(3-monoethoxydimethylsilylpropyl)trisulfide, bis(3-monoethoxydimethylsilylpropyl)disulfide, bis(3-monomethoxydimethylsilylpropyl)tetrasulfide, bis(3-monomethoxydimethylsilylpropyl)trisulfide, bis(3-monomethoxydimethylsilylpropyl)disulfide, bis(2-monoethoxydimethylsilylethyl)tetrasulfide, bis(2-monoethoxydimethylsilylethyl)trisulfide, bis(2-monoethoxydimethylsilylethyl)disulfide, and the like. Particularly preferred among these is bis(3-triethoxysilylpropyl)tetrasulfide.

Examples of thioester-based silane coupling agents include 3-hexanoylthiopropyltriethoxysilane, 3-octanoylthiopropyltriethoxysilane, 3-decanoylthiopropyltriethoxysilane, 3-lauroylthiopropyltriethoxysilane, 2-hexanoylthioethyltriethoxysilane, 2-octanoylthioethyltriethoxysilane, 2-decanoylthioethyltriethoxysilane, 2-lauroylthioethyltriethoxysilane, 3-hexanoylthiopropyltrimethoxysilane, 3-octanoylthiopropyltrimethoxysilane, 3-decanoylthiopropyltrimethoxysilane, 3-lauroylthiopropyltrimethoxysilane, 2-hexanoylthioethyltrimethoxysilane, 2-octanoylthioethyltrimethoxysilane, 2-decanoylthioethyltrimethoxysilane, 2-lauroylthioethyltrimethoxysilane, and the like.

Examples of thiol-based silane coupling agents include 3-mercaptopropyltrimethoxysilane, 3-mercaptopropyltriethoxysilane, 3-mercaptopropylmethyldimethoxysilane, 3-[ethoxybis(3,6,9,12,15-pentoxaoctacosan-1-yloxy)silyl]-1-propanethiol, and the like.

Examples of olefin-based silane coupling agents include dimethoxymethylvinylsilane, vinyltrimethoxysilane, dimethylethoxyvinylsilane, diethoxymethylvinylsilane, triethoxyvinylsilane, vinyltris(2-methoxyethoxy)silane, allyltrimethoxysilane, allyltriethoxysilane, p-styryltrimethoxysilane, 3-(methoxydimethoxydimethylsilyl)propyl acrylate, 3-(trimethoxysilyl)propyl acrylate, 3-[dimethoxy(methyl)silyl]propyl methacrylate, 3-(trimethoxysilyl)propyl methacrylate, 3-[dimethoxy(methyl)silyl]propyl methacrylate, 3-(triethoxysilyl)propyl methacrylate, 3-[tris(trimethylsiloxy)silyl]propyl methacrylate, and the like.

Examples of epoxy-based silane agents include 3-glycidyloxypropyl(dimethoxy)methylsilane, 3-glycidyloxypropyltrimethoxysilane, diethoxy(3-glycidyloxypropyl)methylsilane, triethoxy(3-glycidyloxypropyl)silane, 2-(3,4-epoxycyclohexyl)ethyltrimethoxysilane, and the like. Preferred among these is 3-glycidyloxypropyltrimethoxysilane.

Examples of amino-based silane coupling agents include N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-ethoxysilyl-N-(1,3-dimethylbutylidene)propylamine, N-phenyl-3-aminopropyltrimethoxysilane, N-(vinylbenzyl)-2-aminoethyl-3-aminopropyltrimethoxysilane, and the like. Preferred among these is 3-aminopropyltriethoxysilane.

Examples of alkyl-based silane coupling agents include methyltrimethoxysilane, dimethyldimethoxysilane, trimethylmethoxysilane, methyltriethoxysilane, ethyltrimethoxysilane, n-propyltrimethoxysilane, isobutyltrimethoxysilane, isobutyltriethoxysilane, n-hexyltrimethoxysilane, n-hexyltriethoxysilane, cyclohexylmethyldimethoxysilane, n-octyltriethoxysilane, n-decyltrimethoxysilane, and the like. Preferred among these is methyltriethoxysilane.

Among these silane coupling agents, bis(3-triethoxysilylpropyl)tetrasulfide can be particularly preferably used.

Titanate coupling agents that can be used in combination with the carbon black and/or inorganic filler are not particularly limited, and commercially available products can be suitably used. Examples of such titanate coupling agents include alkoxide-based, chelate-based, and acylate-based titanate coupling agents.

Examples of alkoxide-based titanate coupling agents include tetraisopropyl titanate, tetra-normal butyl titanate, butyl titanate dimer, tetraoctyl titanate, tetra-tertiary butyl titanate, tetrastearyl titanate, and the like. Preferred among these is tetraisopropyl titanate.

Examples of chelate-based titanate coupling agents include titanium acetylacetonate, titanium tetraacetylacetonate, titanium ethylacetoacetate, titanium dodecylbenzenesulfonate compounds, titanium phosphate compounds, titanium octylene glycolate, titanium ethyl acetoacetate, titanium lactate ammonium salt, titanium lactate, titanium ethanol aminate, titanium octylene glycolate, titanium aminoethylaminoethanolate, and the like. Preferred among these is titanium acetylacetonate.

Examples of acylate-based titanate coupling agents include titanium isostearate and the like.

Aluminate coupling agents that can be used in combination with the carbon black and/or inorganic filler are not particularly limited, and commercially available products can be suitably used. Examples of such aluminate coupling agents include 9-octadecenyl acetoacetate aluminum diisopropylate, aluminum secondary butoxide, aluminum trisacetylacetonate, aluminum bisethylacetoacetate monoacetylacetonate, aluminum trisethylacetoacetate, and the like. Preferred among these is 9-octadecenyl acetoacetate aluminum diisopropylate.

Zirconate coupling agents that can be used in combination with the carbon black and/or inorganic filler are not particularly limited, and commercially available products can be suitably used. Examples of such zirconate coupling agents include alkoxide-based, chelate-based, and acylate-based zirconate coupling agents.

Examples of alkoxide-based zirconate coupling agents include normal-propyl zirconate, normal-butyl zirconate, and the like. Preferred among these is normal-butyl zirconate.

Examples of chelate-based zirconate coupling agents include zirconium tetraacetylacetonate, zirconium monoacetylacetonate, zirconium ethylacetoacetate, zirconium lactate ammonium salt, and the like. Preferred among these is zirconium tetraacetylacetonate.

Examples of acylate-based zirconate coupling agents include zirconium stearate, zirconium octylate, and the like. Preferred among these is zirconium stearate.

In the present invention, the silane coupling agents, titanate coupling agents, aluminate coupling agents, and zirconate coupling agents can be used singly or in combination of two or more.

The amount of the silane coupling agent blended in the rubber composition for producing a foam having a high expansion ratio of the present invention is preferably 0.1 to 20 parts by mass, and more preferably 3 to 15 parts by mass, based on 100 parts by mass of the carbon black and/or inorganic filler. When the amount of the silane coupling agent is 0.1 parts by mass or more, the effect of improving the tear strength of the rubber composition can be more suitably expressed. When the amount of the silane coupling agent is 20 parts by mass or less, the cost of the rubber composition is reduced, and economic efficiency is improved.

## 2. High-Expansion-Ratio Foam

The present invention includes a high-expansion-ratio foam foamed from the rubber composition for producing a foam having a high expansion ratio. The foam also includes a molded product obtained by molding the foam.

The high-expansion-ratio foam in the rubber composition for producing a foam having a high expansion ratio of the present invention is defined as having an expansion ratio index larger than that, represented by formula 2, of a foam produced from a composition containing components (a), (b), and (d), and not containing component (c) (hereinafter, "foam X"). Further, in the present specification, the high-expansion-ratio foam is defined as having an expansion ratio index of 101 or more. The expansion ratio index is a value calculated based on the following formulas 1 and 2. Preferred among high-expansion-ratio foams are those having an expansion ratio index of 103 or more, more preferably 105 or

more, and particularly preferably 110 or more. The amount of component (b) in the foam produced from the composition comprising components (a), (b), (c), and (d) (hereinafter, "the rubber composition of the present invention") is the same as that of foam X.

Formula 1: Expansion ratio

$$= \text{specific gravity before foam molding}/\text{specific gravity after foam molding} \times 100$$

Formula 2: Expansion ratio index

$$= (\text{expansion ratio of foam produced from rubber composition of present invention}) \times 100/(\text{expansion ratio of foam X})$$

For example, when component (a) is butyl rubber, the expansion ratio index of the high-expansion-ratio foam is 120 or more, preferably 130 or more, and particularly preferably 140 or more.

For example, when component (a) is a combination of natural rubber and butadiene rubber, the expansion ratio index of the high-expansion-ratio foam is 103 or more, preferably 105 or more, and particularly preferably 110 or more.

For example, when component (a) is ethylene-propylene-diene terpolymer rubber (EPDM), the expansion ratio index of the high-expansion-ratio foam is 103 or more, preferably 105 or more, and particularly preferably 110.

3. Tire, Acoustic Member, Sealing Material, Hose, Belt, Wire Covering, and Thermal Insulation Material

Further, the present invention includes a tire, an acoustic member, a sealing material, a hose, a belt, a wire covering, and a thermal insulation material, all of which are produced by using the rubber composition for producing a foam having a high expansion ratio and the high-expansion-ratio foam.

Examples of the tire of the present invention include pneumatic tires (radial tires, bias tires, etc.), solid tires, and the like.

Tire applications are not particularly limited, and examples include passenger car tires, heavy-duty tires, motorcycle tires, studless tires, and the like. Preferred among these applications are passenger car tires or studless tires.

The shape, structure, size, and material of the tire of the present invention are not particularly limited, and can be suitably selected depending on the intended purpose.

In the tire of the present invention, the above rubber composition is used particularly for at least one member selected from tread, sidewall, bead area, belt, carcass, and shoulder portions.

Among these, it is preferable that a tire tread or sidewall portion of a pneumatic tire is formed using the rubber composition, and it is particularly preferable that a tire tread portion of a pneumatic tire is formed using the rubber composition.

The "tread" is a portion that has a tread pattern and comes into direct contact with the road surface. The tread refers to a tire casing portion for protecting the carcass and preventing wear and flaws, and refers to a cap tread that constitutes the grounding part of a tire and/or to a base tread that is disposed inside the cap tread.

The "sidewall" refers to, for example, a portion from the lower side of a shoulder portion to a bead portion of a pneumatic radial-ply tire. Sidewall portions protect the carcass and are bent the most when the vehicle drives.

The "bead area" portions function to anchor both ends of carcass cords and simultaneously hold the tire to the rim. Beads are composed of bundles of high carbon steel.

The "belt" refers to a reinforcing band disposed between the carcass and the tread of a radial structure in the circumferential direction. The belt tightens the carcass like a hoop of a barrel to enhance the rigidity of the tread.

The "carcass" refers to a cord layer portion that forms the framework of the tire. The carcass plays a role in bearing the load, impact, and filled air pressure applied to the tire.

The "shoulder" refers to a shoulder portion of the tire. Shoulder portions play a role in protecting the carcass.

The tire of the present invention can be produced by methods known in the field of tires. The tire may be filled with ordinary air, or air having an adjusted oxygen partial pressure; or an inert gas, such as nitrogen, argon, or helium.

Further, examples of the acoustic member of the present invention include speaker edges, sound absorbers, sound insulators, vibration isolators, vibration-damping materials, and the like. Examples of the sealing material of the present invention include rubber packing, oil seals, water seals, jointing materials, weather strips, glass run channels,

and the like. Examples of the hose of the present invention include radiator hoses, water hoses, concrete transport hoses, and the like. Examples of the belt of the present invention include transmission belts, conveyor belts, V-belts, and the like. Examples of the wire covering of the present invention include coverings for power lines, and the like. Examples of the thermal insulation material of the present invention include thermal insulation materials for piping for transporting refrigerants, heat media, etc., and thermal insulation materials for floors, walls, etc.

When the rubber composition for producing a foam having a high expansion ratio of the present invention is used in a transmission belt, particularly a conveyor belt, such a belt can be produced, for example, by closely adhering the rubber composition to a reinforcing material, followed by vulcanization. Specifically, the rubber composition is extruded to produce sheet-like cover rubber layers, a reinforcing material is sandwiched between the cover rubber layers from above and below, and this belt molded product is set in a mold and vulcanized at a predetermined temperature and pressure for a predetermined period of time. As the reinforcing material, a material generally used for conveyor belts can be suitably selected and used, depending on the application of the conveyor belt, in consideration of size and other factors.

4. Expansion Ratio Improver

The present invention further includes an expansion ratio improver (expansion ratio improver for rubber). In the present specification, the expansion ratio improver is defined as an agent that can act on the rubber composition to improve the expansion ratio, unlike a foaming aid, which acts on the chemical foaming agent (reduces the decomposition temperature of the chemical foaming agent) to thereby promote foaming.

More specifically, the expansion ratio improver (expansion ratio improver for rubber) of the present invention is an expansion ratio improver for tires, an expansion ratio improver for acoustic members, an expansion ratio improver for sealing materials, an expansion ratio improver for hoses, an expansion ratio improver for belts, an expansion ratio improver for wire coverings, or an expansion ratio improver for thermal insulation materials, and contains component (c) described above.

Component (c) contained in the expansion ratio improver of the present invention also includes tautomers of compound (1) described above.

The amount of the expansion ratio improver of the present invention blended is generally 0.01 to 50 parts by mass, preferably 0.05 to 40 parts by mass, more preferably 0.1 to 30 parts by mass, and even more preferably 0.5 to 10 parts by mass, based on 100 parts by mass of component (a) in the rubber composition. Because the amount of the additive for rubber (the expansion ratio improver) of the present invention blended is 0.01 parts by mass or more based on 100 parts by mass of component (a), the expansion ratio of the rubber composition can be improved. On the other hand, the amount of the additive for rubber (the expansion ratio improver) being set to 50 parts by mass or less based on 100 parts by mass of component (a) is economically preferred.

The expansion ratio improver of the present invention is preferably component (c) itself, but may contain, as appropriate, other components such as fillers.

Regarding the blending ratio of component (c) in the expansion ratio improver of the present invention and component (b), the ratio of the expansion ratio improver of the present invention is preferably 0.1 to 80 parts by mass, more preferably 1 to 75 parts by mass, even more preferably 3 to 70 parts by mass, and particularly preferably 5 to 65 parts by mass, based on the total mass of component (c) in the expansion ratio improver of the present invention and component (b), which is taken as 100 parts by mass.

The addition of the expansion ratio improver of the present invention to the rubber component can improve the expansion ratio of the rubber composition.

5. Method for Improving Expansion Ratio of Foam

The present invention includes a method for improving the expansion ratio of a foam.

The method for improving the expansion ratio of a foam of the present invention comprises mixing a rubber component, a chemical foaming agent, and a compound represented by the following formula (1) or a salt thereof (compound (1)):

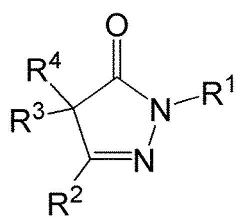

(1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

In the method for improving the expansion ratio of a foam of the present invention, the rubber component, the chemical foaming agent, and compound (1) may be simply mixed, or after the rubber component and compound (1) are mixed, the chemical foaming agent may be mixed. That is, the rubber component, the chemical foaming agent, and compound (1) may be mixed in any order.

6. Method for Producing High-Expansion-Ratio Foam

The method for producing a high-expansion-ratio foam of the present invention is, for example, a method of foaming the rubber composition for producing a foam having a high expansion ratio of the present invention, which comprises components (a), (b), (c), and (d) .

As the method for producing the rubber composition for producing a foam having a high expansion ratio of the present invention, components (a), (b), (c), and (d) may be mixed, and other ingredients may be mixed, as necessary. The order of blending may be suitably set.

Examples include a method comprising

step (A) of mixing a raw material component containing components (a), (b), and (c), and step (B) of mixing the mixture obtained in step (A) with component (d);
a method comprising step (A) of mixing a raw material component containing components (a) and (b), and step (B) of mixing the mixture obtained in step (A) with components (c) and (d) ;
a method comprising step (A) of mixing a raw material component containing components (a) and (c), and step (B) of mixing the mixture obtained in step (A) with components (b) and (d); and the like.

Preferred among these methods is a method comprising step (A) of kneading a raw material component containing components (a) and (c), and step (B) of mixing the mixture obtained in step (A) with components (b) and (d).

Then, the method further comprises step (C) of foaming the obtained rubber composition, whereby a higher-expansion ratio foam, for example, a high-expansion-ratio foam having an expansion ratio index of 103 or more, can be obtained.

6.1. Step (A)

Step (A) is step (A1) of mixing a raw material component containing components (a), (b), and (c), step (A2) of mixing a raw material component containing components (a) and (b), or step (A3) of mixing a raw material component containing components (a) and (c). Step (A) is preferably step (A3) of mixing a raw material component containing components (a) and (c) .

In step (A), the other ingredients mentioned above may be further blended. In the present specification, the term "mixing" includes not only the mere act of mixing, but also the act of so-called "kneading."

In step (A), a raw material component containing component (a) and at least one member selected from the group consisting of components (b) and (c) is mixed. In the method of mixing these components, the entire amount of each component may be mixed at once, or each component may be divided and mixed according to the purpose such as viscosity adjustment. The mixing operation may be repeated to uniformly disperse each component. Alternatively, filler masterbatch rubber may be used, in which a filler is added to rubber in advance by wet and/or dry mixing methods.

Further, when carbon black and/or an inorganic filler are added and mixed in the high-expansion-ratio foam of the present invention, other mixing methods in step (A) include a two-step mixing method comprising step (A-1) of mixing component (a) with at least one member selected from the group consisting of components (b) and (c), and step (A-2) of mixing the mixture obtained in step (A-1) with a raw material component containing carbon black and/or an inorganic filler.

The temperature during mixing of the rubber composition in step (A) is not particularly limited. For example, the upper limit of the temperature of the rubber composition is preferably 100 to 190°C, more preferably 110 to 175°C, and even more preferably 120 to 170°C.

The mixing time in step (A) is not particularly limited, and is preferably, for example, 10 seconds to 20 minutes, more preferably 30 seconds to 10 minutes, and even more preferably 1 minute to 8 minutes.

Regarding the temperature during mixing of components (a) and (c) in step (A-1), the upper limit of the temperature of the rubber composition is preferably 60 to 190°C, more preferably 70 to 160°C, and even more preferably 80 to 150°C. This is because the reaction does not proceed at a mixing temperature lower than 60°C, and rubber degradation progresses at a mixing temperature of 190°C or more, or more than 190°C.

The mixing time in step (A-1) is preferably 10 seconds to 20 minutes, more preferably 30 seconds to 10 minutes, and even more preferably 60 seconds to 7 minutes. By setting the mixing time to 10 seconds or more, the reaction can

be sufficiently advanced. On the other hand, the mixing time is set within 20 minutes, which is superior in terms of productivity.

The temperature during mixing of the mixture obtained in step (A-1) with carbon black and/or an inorganic filler in step (A-2) is not particularly limited. For example, the upper limit of the temperature of the mixture is preferably 100 to 190°C, more preferably 110 to 175°C, and even more preferably 130 to 170°C.

The mixing time in step (A-2) is not particularly limited, and is preferably, for example, 10 seconds to 20 minutes, more preferably 30 seconds to 10 minutes, and even more preferably 1 minute to 8 minutes.

6.2. Step (B)

Step (B) is to mix the mixture obtained in step (A) with component (d). When the mixture is obtained in step (A1), this step is to mix component (d). When the mixture is obtained in step (A2), this step is to mix components (c) and (d). When the mixture is obtained in step (A3), this step is to mix components (b) and (d).

In step (B), a vulcanization accelerator etc. may be further blended, as necessary.

Step (B) can be performed under heating conditions. The heating temperature in this step is not particularly limited, and is preferably, for example, 60 to 140°C, more preferably 80 to 120°C, and even more preferably 90 to 120°C.

The mixing time is not particularly limited, and is preferably, for example, 10 seconds to 20 minutes, more preferably 30 seconds to 10 minutes, and even more preferably 60 seconds to 5 minutes.

When proceeding from step (A) to step (B), it is preferable to lower the temperature by 30°C from the temperature at the time when the previous step is completed, before proceeding to the next step (B).

In the method for producing a high-expansion-ratio foam of the present invention, various ingredients, such as stearic acid, zinc oxide, and antiaging agents, which can be generally blended in rubber compositions, can be added, if necessary, in step (A) or (B).

The above ingredients may be added in either step (A) or (B), or may be added separately in steps (A) and (B).

6.3. Step (C): Vulcanization (and/or Crosslinking) and Foaming Step

Step (C) is to perform vulcanization (and/or crosslinking) and foaming by heating the mixture obtained in step (B) .

The heating temperature may be equal to or higher than the decomposition temperature of mixed component (b), and is preferably, for example, 50 to 300°C, more preferably 80 to 250°C, and even more preferably 100 to 200°C.

The heating time is not particularly limited, and is preferably, for example, 5 seconds to 24 hours, more preferably 10 seconds to 12 hours, and even more preferably 5 minutes to 3 hours.

Moreover, step (C) may be performed under pressure. The pressure applied is not particularly limited, and is preferably, for example, 0.1 to 300 $kgf/cm^2$, more preferably 10 to 250 $kgf/cm^2$, and even more preferably 50 to 200 $kgf/cm^2$.

The pressurization time is not particularly limited, and is preferably, for example, 5 seconds to 24 hours, more preferably 10 seconds to 12 hours, and even more preferably 5 minutes to 3 hours. Step (C) is preferably performed by filling a mold or the like with the mixture obtained in step (B). A pressure press or a vulcanizing can may be used.

The method preferably comprises step (A) of kneading a raw material component containing component (a) and component (c), and step (B) of mixing the mixture obtained in step (A) with components (b) and (d). This is because the rubber composition obtained by this method can be foamed to thereby obtain a higher-expansion ratio foam, for example, a high-expansion-ratio foam having an expansion ratio index of 103 or more.

6.4. Tire

Tires using the high-expansion-ratio foam of the present invention are not particularly limited, and can be produced, for example, in the following manner.

First, the mixing operations in steps (A) and (B) are performed using a Banbury mixer, a roll, an intensive mixer, a kneader, a single-screw extruder, a twin-screw extruder, or the like. After that, the resultant is extruded and processed in an extrusion step to be molded as, for example, a tread member or a sidewall member. Then, the molded member is adhesion-molded by a usual method on a tire molding machine to form a green tire. The green tire is subjected to the heating and pressurizing operations in step (C) in a vulcanizing machine, whereby a tire produced by using the high-expansion-ratio foam can be obtained.

The embodiments of the present invention are described above; however, the present invention is not limited to these examples. Needless to say, the present invention can be carried out in various forms without departing from the gist of the present invention.

Examples

The embodiments of the present invention are described in more detail below based on Examples; however, the present invention is not limited thereto.

Examples 1 to 7 and Comparative Examples 1 and 2: Production of High-Expansion-Ratio Foam

The components shown in step (A) of Table 1 below were mixed at the shown ratio (parts by mass) in a Banbury mixer. After the mixture was cured until its temperature became 60°C or lower, the components shown in step (B) of Table 1 were put at the shown ratio (parts by mass). The mixture was mixed so that its maximum temperature became 70°C or lower, followed by heating to 160°C, thereby forming a high-expansion-ratio foam.

Examples 1 to 7 and Comparative Examples 1 and 2: Expansion Ratio Index

Regarding the expansion ratio, the specific gravity was measured before and after foam molding using an electronic hydrometer (MDS-300, produced by Alfa Mirage), and the expansion ratio was calculated.

For comparison, foams were prepared using the same formulation and the same production process as in each Example, except that component (c) was not added (Comparative Examples 1 and 2), their expansion ratio was expressed as an index set to 100, and the expansion ratio index was calculated based on the following formula.

The larger the value of expansion ratio index, the higher the expansion ratio and the better the foam.

Formula: expansion ratio = specific gravity before foam molding/specific gravity after foam molding × 100

Formula: expansion ratio index = (expansion ratio of each of Examples 1 to 6) × 100/(expansion ratio of Comparative Example 1)

Formula: expansion ratio index = (expansion ratio of Example 7) × 100/(expansion ratio of Comparative Example 2)

Table 1

| Step | Component | Example | | | | | | | Comp. Example | |
|---|---|---|---|---|---|---|---|---|---|---|
| | | 1 | 2 | 3 | 4 | 5 | 6 | 7 | 1 | 2 |
| A | Butyl rubber*1 | 100 | 100 | 100 | 100 | 100 | 100 | 100 | 100 | 100 |
| | Carbon black A*2 | 60 | 60 | 60 | 60 | 60 | 60 | 60 | 60 | 60 |
| | Wax*3 | 2 | 2 | 2 | 2 | 2 | 2 | 2 | 2 | 2 |
| | Stearic acid*4 | 2 | 2 | 2 | 2 | 2 | 2 | 2 | 2 | 2 |
| | Oil*5 | 23 | 23 | 23 | 23 | 23 | 23 | 23 | 23 | 23 |
| | Zinc oxide*6 | 5 | 5 | 5 | 5 | 5 | 5 | 5 | 5 | 5 |
| | Compound A*7 | 1 | 2 | 3 | | | | 1 | | |

(continued)

| Step | Component | Example | | | | | | | Comp. Example | |
|---|---|---|---|---|---|---|---|---|---|---|
| | | 1 | 2 | 3 | 4 | 5 | 6 | 7 | 1 | 2 |
| B | Vulcanization accelerator A*8 | 1 | 1 | 1 | 1 | 1 | 1 | 1 | 1 | 1 |
| | Vulcanization accelerator B*9 | 1 | 1 | 1 | 1 | 1 | 1 | 1 | 1 | 1 |
| | Sulfur*10 | 1.5 | 1.5 | 1.5 | 1.5 | 1.5 | 1.5 | 1.5 | 1.5 | 1.5 |
| | Foaming agent A*11 | 5 | 5 | 5 | 5 | 5 | 5 | 5 | 5 | 5 |
| | Foaming aid*12 | 7 | 7 | 7 | 7 | 7 | 7 | 7 | 7 | 7 |
| | Foaming agent B*13 | | | | | | | 5 | | 5 |
| | Compound A*7 | | | | 1 | 2 | 3 | | | |
| Expansion ratio index | | 153 | 157 | 193 | 126 | 183 | 145 | 139 | 100 | 100 |

The details of each component in Table 1 are as described below.

*1: component (a): butyl rubber; JSR BUTYL 365, produced by JSR
*2: carbon black A; Seast SO (N550), produced by Tokai Carbon Co., Ltd.
*3: wax; Sunnoc, produced by Ouchi Shinko Chemical Industrial Co., Ltd.
*4: stearic acid; Stearic Acid 50S, produced by New Japan Chemical Co., Ltd.
*5: oil; Diana Process Oil NR-26, produced by Idemitsu Showa Shell
*6: zinc oxide; Type 1, produced by Sakai Chemical Industry Co., Ltd.
*7: component (c): compound A; 3-methyl-5-pyrazolone, produced by Otsuka Chemical Co., Ltd.
*8: vulcanization accelerator A; Nocceler M-P, produced by Ouchi Shinko Chemical Industrial Co., Ltd.
*9: vulcanization accelerator B; Nocceler TT-P, produced by Ouchi Shinko Chemical Industrial Co., Ltd.
*10: component (d): sulfur; HK200-5, produced by Hosoi Chemical Industry Co., Ltd.
*11: component (b): foaming agent A; Unifoam AZ VI-40 (azodicarbonamide, median diameter: 3.2±0.7 μm), produced by Otsuka Chemical Co., Ltd.
*12: component (e): foaming aid; Unifoam AZ 01, produced by Otsuka Chemical Co., Ltd.
*13: component (b): foaming agent B; Unifoam AZ P-5 (sodium bicarbonate), produced by Otsuka Chemical Co., Ltd.

Examples 8 to 18 and Comparative Examples 3 and 4: Production of High-Expansion-Ratio Foam

The components shown in step (A) of Table 2 below were mixed at the shown ratio (parts by mass) in a Banbury mixer. After the mixture was cured until its temperature became 60°C or lower, the components shown in step (B) of Table 2 were put at the shown ratio (parts by mass). The mixture was mixed so that its maximum temperature became 70°C or lower, followed by heating to 160°C, thereby forming a high-expansion-ratio foam.

Examples 8 to 18 and Comparative Examples 3 and 4: Expansion Ratio Index

Regarding the expansion ratio, the specific gravity was measured before and after foam molding using an electronic hydrometer (MDS-300, produced by Alfa Mirage), and the expansion ratio was calculated.
For comparison, foams were prepared using the same formulation and the same production process as in each Example, except that component (c) was not added (Comparative Examples 3 and 4), their expansion ratio was expressed as an index set to 100, and the expansion ratio index was calculated based on the following formula.
The larger the value of expansion ratio index, the higher the expansion ratio and the better the foam.

Formula: expansion ratio
= specific gravity before foam molding/specific gravity after foam molding × 100

Formula: expansion ratio index

$$\text{expansion ratio index} = (\text{expansion ratio of each of Examples 8 to 15}) \times 100/(\text{expansion ratio of Comparative Example 3})$$

Formula: expansion ratio index

$$\text{expansion ratio index} = (\text{expansion ratio of each of Examples 16 to 18}) \times 100/(\text{expansion ratio of Comparative Example 4})$$

Table 2

| Step | Component | Example | | | | | | | | | | | Comp. Example | |
|---|---|---|---|---|---|---|---|---|---|---|---|---|---|---|
| | | 8 | 9 | 10 | 11 | 12 | 13 | 14 | 15 | 16 | 17 | 18 | 3 | 4 |
| A | Natural rubber*14 | 50 | 50 | 50 | 50 | 50 | 50 | 50 | 50 | 50 | 50 | 50 | 50 | 50 |
| | Butadiene rubber*15 | 50 | 50 | 50 | 50 | 50 | 50 | 50 | 50 | 50 | 50 | 50 | 50 | 50 |
| | Silica*16 | 35 | 35 | 35 | 35 | 35 | 35 | 35 | 35 | 35 | 35 | 35 | 35 | 35 |
| | Carbon black B*17 | 20 | 20 | 20 | 20 | 20 | 20 | 20 | 20 | 20 | 20 | 20 | 20 | 20 |
| | Silane coupling agent*18 | 2.8 | 2.8 | 2.8 | 2.8 | 2.8 | 2.8 | 2.8 | 2.8 | 2.8 | 2.8 | 2.8 | 2.8 | 2.8 |
| | Antiaging agent*19 | 1 | 1 | 1 | 1 | 1 | 1 | 1 | 1 | 1 | 1 | 1 | 1 | 1 |
| | Wax*3 | 2 | 2 | 2 | 2 | 2 | 2 | 2 | 2 | 2 | 2 | 2 | 2 | 2 |
| | Oil*5 | 10 | 10 | 10 | 10 | 10 | 10 | 10 | 10 | 10 | 10 | 10 | 10 | 10 |
| | Stearic acid*4 | 2 | 2 | 2 | 2 | 2 | 2 | 2 | 2 | 2 | 2 | 2 | 2 | 2 |
| | Zinc oxide*6 | 3.5 | 3.5 | 3.5 | 3.5 | 3.5 | 3.5 | 3.5 | 3.5 | 3.5 | 3.5 | 3.5 | 3.5 | 3.5 |
| | Compound A*7 | | | | | | 0.5 | 2 | 4 | 2 | 4 | 6 | | |
| B | Vulcanization accelerator A*8 | 0.8 | 0.8 | 0.8 | 0.8 | 0.8 | 0.8 | 0.8 | 0.8 | 0.8 | 0.8 | 0.8 | 0.8 | 0.8 |
| | Vulcanization accelerator C*20 | 1 | 1 | 1 | 1 | 1 | 1 | 1 | 1 | 1 | 1 | 1 | 1 | 1 |
| | Sulfur*10 | 1 | 1 | 1 | 1 | 1 | 1 | 1 | 1 | 1 | 1 | 1 | 1 | 1 |
| | Foaming agent A*11 | 2.75 | 2.75 | 2.75 | 2.75 | 2.75 | 2.75 | 2.75 | 2.75 | | | | 2.75 | |
| | Foaming agent B*13 | | | | | | | | | 5 | 5 | 5 | | 5 |
| | Foaming aid*12 | 2.75 | 2.75 | 2.75 | 2.75 | 2.75 | 2.75 | 2.75 | 2.75 | | | | 2.75 | |
| | Compound A*7 | 0.5 | 1 | 2 | 3 | 4 | | | | | | | | |
| Expansion ratio index | | 101 | 103 | 107 | 116 | 116 | 107 | 110 | 125 | 104 | 105 | 105 | 100 | 100 |

The details of each component in Table 2 are as described below.

*14: component (a): natural rubber; TSR-20, produced by Guangken Rubber
*15: component (a): butadiene rubber; UBEPOL BR 150B, produced by UBE Corporation
*16: silica; Nipsil AQ, produced by Tosoh Corporation
*17: carbon black B; Seast 7HM (N234), produced by Tokai Carbon Co., Ltd.
*18: silane coupling agent; Si69, produced by Evonic Industries
*19: antiaging agent; Nocrac 6C, produced by Ouchi Shinko Chemical Industrial Co., Ltd.
*20: vulcanization accelerator C; Nocceler CZ-G, produced by Ouchi Shinko Chemical Industrial Co., Ltd.

Examples 19 to 23 and Comparative Examples 5 to 8: Production of High-Expansion-Ratio Foam

The components shown in step (A) of Table 3 below were mixed at the shown ratio (parts by mass) in a Banbury mixer. After the mixture was cured until its temperature became 60°C or lower, the components shown in step (B) of Table 3 were put at the shown ratio (parts by mass). The mixture was mixed so that its maximum temperature became 70°C or lower, followed by heating to 200°C, thereby forming a high-expansion-ratio foam.

Examples 19 to 23 and Comparative Examples 5 to 8: Expansion Ratio Index

Regarding the expansion ratio, the specific gravity was measured before and after foam molding using an electronic hydrometer (MDS-300, produced by Alfa Mirage), and the expansion ratio was calculated.
For comparison, foams were prepared using the same formulation and the same production process as in each Example, except that component (c) was not added (Comparative Examples 5 to 8), their expansion ratio was expressed as an index set to 100, and the expansion ratio index was calculated based on the following formula.
The larger the value of expansion ratio index, the higher the expansion ratio and the better the foam.

Formula: expansion ratio = specific gravity before foam molding/specific gravity after foam molding × 100

Formula: expansion ratio index = (expansion ratio of Example 19) × 100/(expansion ratio of Comparative Example 5)

Formula: expansion ratio index = (expansion ratio of each of Examples 20 and 21) × 100/(expansion ratio of Comparative Example 6)

Formula: expansion ratio index = (expansion ratio of Example 22) × 100/(expansion ratio of Comparative Example 7)

Formula: expansion ratio index = (expansion ratio of Example 23) × 100/(expansion ratio of Comparative Example 8)

Table 3

| Step | Component | Example | | | | | Comp. Example | | | |
|---|---|---|---|---|---|---|---|---|---|---|
| | | 19 | 20 | 21 | 22 | 23 | 5 | 6 | 7 | 8 |
| A | EPDM*21 | 100 | 100 | 100 | 100 | 100 | 100 | 100 | 100 | 100 |
| | Carbon black C*22 | 80 | 80 | 80 | 80 | 80 | 80 | 80 | 80 | 80 |
| | Calcium carbonate*23 | 15 | 15 | 15 | 15 | 15 | 15 | 15 | 15 | 15 |
| | Oil*5 | 45 | 45 | 45 | 45 | 45 | 45 | 45 | 45 | 45 |
| | Stearic acid*4 | 2 | 2 | 2 | 2 | 2 | 2 | 2 | 2 | 2 |
| | Zinc oxide*6 | 5 | 5 | 5 | 5 | 5 | 5 | 5 | 5 | 5 |
| | Compound A*7 | 1 | 1 | | 1 | 1 | | | | |
| B | Vulcanization accelerator D*24 | 1 | 1 | 1 | 1 | 1 | 1 | 1 | 1 | 1 |
| | Vulcanization accelerator E*25 | 1.5 | 1.5 | 1.5 | 1.5 | 1.5 | 1.5 | 1.5 | 1.5 | 1.5 |
| | Vulcanization accelerator F*26 | 0.5 | 0.5 | 0.5 | 0.5 | 0.5 | 0.5 | 0.5 | 0.5 | 0.5 |
| | Vulcanization accelerator G*27 | 1 | 1 | 1 | 1 | 1 | 1 | 1 | 1 | 1 |
| | Sulfur*10 | 0.5 | 0.5 | 0.5 | 0.5 | 0.5 | 0.5 | 0.5 | 0.5 | 0.5 |
| | Foaming agent A*11 | 6 | 6 | 6 | | | 6 | 6 | | |
| | Foaming agent B*13 | | | | 6 | | | | 6 | |
| | Foaming agent C*28 | | | | | 6 | | | | 6 |
| | Foaming aid*12 | | 3 | 3 | | | | 3 | | |
| | Compound A*7 | | | 1 | | | | | | |
| Expansion ratio index | | 108 | 126 | 124 | 101 | 125 | 100 | 100 | 100 | 100 |

The details of each component in Table 3 are as described below.

*21: component (a): EPDM; Esprene 505A, produced by Sumitomo Chemical Co., Ltd.
*22: carbon black C; #50, produced by Asahi Carbon Co., Ltd.
*23: light calcium carbonate, produced by Toyo Denka Kogyo Co., Ltd.
*24: vulcanization accelerator D; Nocceler M, produced by Ouchi Shinko Chemical Industrial Co., Ltd.
*25: vulcanization accelerator E; Nocceler BZ-P, produced by Ouchi Shinko Chemical Industrial Co., Ltd.
*26: vulcanization accelerator F; Nocceler TET-G, produced by Ouchi Shinko Chemical Industrial Co., Ltd.
*27: vulcanization accelerator G; Nocceler TRA, produced by Ouchi Shinko Chemical Industrial Co., Ltd.
*28: component (b): foaming agent C; Unifoam AZ 100 (p,p'-oxybisbenzenesulfonyl hydrazide), produced by Otsuka Chemical Co., Ltd.

Examples 24 to 27 and Comparative Examples 9 and 10: Production of High-Expansion-Ratio Foam

The components shown in step (A) of Table 4 below were mixed at the shown ratio (parts by mass) in a Banbury mixer. After the mixture was cured until its temperature became 60°C or lower, the components shown in step (B) of Table 4 were put at the shown ratio (parts by mass). The mixture was mixed so that its maximum temperature became 70°C or lower, followed by heating to 160°C, thereby forming a high-expansion-ratio foam.

Examples 24 to 27 and Comparative Examples 9 and 10: Expansion Ratio Index

Regarding the expansion ratio, the specific gravity was measured before and after foam molding using an electronic hydrometer (MDS-300, produced by Alfa Mirage), and the expansion ratio was calculated.
For comparison, foams were prepared using the same formulation and the same production process as in each Example, except that component (c) was not added (Comparative Examples 9 and 10), their expansion ratio was

expressed as an index set to 100, and the expansion ratio index was calculated based on the following formula.

The larger the value of expansion ratio index, the higher the expansion ratio and the better the foam.

```
Formula: expansion ratio
          = specific gravity before foam molding/specific gravity
after foam molding × 100
```

```
Formula: expansion ratio index
          = (expansion ratio of each of Examples 24 to 26) ×
100/(expansion ratio of Comparative Example 9)
```

```
Formula: expansion ratio index
          = (expansion ratio of Example 27) × 100/(expansion
ratio of Comparative Example 10)
```

Table 4

| Step | Component | Example | | | | Comp. Example | |
|---|---|---|---|---|---|---|---|
| | | 24 | 25 | 26 | 27 | 9 | 10 |
| A | Natural rubber*14 | 50 | 50 | 50 | 50 | 50 | 50 |
| | Butadiene rubber*15 | 50 | 50 | 50 | 50 | 50 | 50 |
| | Silica*16 | 35 | 35 | 35 | 35 | 35 | 35 |
| | Carbon black B*17 | 20 | 20 | 20 | 20 | 20 | 20 |
| | Silane coupling agent*18 | 2.8 | 2.8 | 2.8 | 2.8 | 2.8 | 2.8 |
| | Antiaging agent*19 | 1 | 1 | 1 | 1 | 1 | 1 |
| | Wax*3 | 2 | 2 | 2 | 2 | 2 | 2 |
| | Oil*5 | 10 | 10 | 10 | 10 | 10 | 10 |
| | Stearic acid*4 | 2 | 2 | 2 | 2 | 2 | 2 |
| | Zinc oxide*6 | 3.5 | 3.5 | 3.5 | 3.5 | 3.5 | 3.5 |
| | Compound A*7 | 3 | 4 | 5 | 4 | | |
| B | Vulcanization accelerator A*8 | 0.8 | 0.8 | 0.8 | 0.8 | 0.8 | 0.8 |
| | Vulcanization accelerator C*20 | 1 | 1 | 1 | 1 | 1 | 1 |
| | Sulfur*10 | 1 | 1 | 1 | 1 | 1 | 1 |
| | Foaming agent D*29 | 3 | 3 | 3 | | 3 | |
| | Foaming aid*12 | 3 | 3 | 3 | 3 | 3 | 3 |
| | Foaming agent A*11 | | | | 3 | | 3 |
| | Expansion ratio index | 113 | 119 | 125 | 118 | 100 | 100 |

The details of each component in Table 4 are as described below.

*29: component (b): foaming agent D; Celmike A (dinitrosopentamethylenetetramine), produced by Sankyo Kasei Co., Ltd.

Examples 28 to 32 and Comparative Examples 11 and 12: Production of High-Expansion-Ratio Foam

The components shown in step (A) of Table 5 below were mixed at the shown ratio (parts by mass) in a Banbury mixer. After the mixture was cured until its temperature became 60°C or lower, the components shown in step (B) of Table 5 were put at the shown ratio (parts by mass). The mixture was mixed so that its maximum temperature became 70°C or lower, followed by heating to 200°C, thereby forming a high-expansion-ratio foam.

Examples 28 to 32 and Comparative Examples 11 and 12: Expansion Ratio Index

Regarding the expansion ratio, the specific gravity was measured before and after foam molding using an electronic hydrometer (MDS-300, produced by Alfa Mirage), and the expansion ratio was calculated.
For comparison, foams were prepared using the same formulation and the same production process as in each Example, except that component (c) was not added (Comparative Examples 11 and 12), their expansion ratio was expressed as an index set to 100, and the expansion ratio index was calculated based on the following formula.
The larger the value of expansion ratio index, the higher the expansion ratio and the better the foam.

Expansion ratio = specific gravity before foam molding/specific gravity after foam molding × 100

Expansion ratio index = (expansion ratio of Examples 28 to 30)/(expansion ratio of Comparative Example 11) × 100

Expansion ratio index = (expansion ratio of Examples 31 and 32)/(expansion ratio of Comparative Example 12) × 100

Examples 28 to 32 and Comparative Examples 11 and 12: Average Cell Diameter Index

The average cell diameter was measured according to ASTM D2842-69.
For comparison, foams were prepared using the same formulation and the same production process as in each Example, except that component (c) was not added (Comparative Examples 11 and 12), their average cell diameter was expressed as an index set to 100, and the average cell diameter index was calculated based on the following formula.
The smaller the value of average cell diameter index, the denser the cell diameter and the better the foam.

Average cell diameter index = (average cell diameter of Examples 28 to 30)/(average cell diameter of Comparative Example 11) × 100

Average cell diameter index = (average cell diameter of Examples 31 and 32)/(average cell diameter of Comparative Example 12) × 100

Examples 28 to 32 and Comparative Examples 11 and 12: Tensile Strength Index

The tensile strength was measured according to JIS K 6251.
For comparison, foams were prepared using the same formulation and the same production process as in each

Example, except that component (c) was not added (Comparative Examples 11 and 12), their tensile strength was expressed as an index set to 100, and the tensile strength index was calculated based on the following formula.

The larger the value of tensile strength index, the higher the tensile strength and the better the foam.

$$\text{Tensile strength index} = (\text{tensile strength of Examples 28 to 30})/(\text{tensile strength of Comparative Example 11}) \times 100$$

$$\text{Tensile strength index} = (\text{tensile strength of Examples 31 and 32})/(\text{tensile strength of Comparative Example 12}) \times 100$$

Table 5

| Step | Component | Comp. Ex. | Example | | | Comp. Ex. | Example | |
|---|---|---|---|---|---|---|---|---|
| | | 11 | 28 | 29 | 30 | 12 | 31 | 32 |
| A | EPDM*21 | 100 | 100 | 100 | 100 | 100 | 100 | 100 |
| | Carbon black*22 | 80 | 80 | 80 | 80 | 80 | 80 | 80 |
| | Calcium carbonate*23 | 15 | 15 | 15 | 15 | 15 | 15 | 15 |
| | Oil*30 | 45 | 45 | 45 | 45 | 45 | 45 | 45 |
| | Stearic acid*4 | 2 | 2 | 2 | 2 | 2 | 2 | 2 |
| | Zinc oxide*6 | 5 | 5 | 5 | 5 | 5 | 5 | 5 |
| | Compound A*7 | - | **0.3** | **0.5** | **1** | - | **0.5** | **1** |
| B | Vulcanization accelerator D*24 | 1 | 1 | 1 | 1 | 1 | 1 | 1 |
| | Vulcanization accelerator E*25 | 1.5 | 1.5 | 1.5 | 1.5 | 1.5 | 1.5 | 1.5 |
| | Vulcanization accelerator F*26 | 0.5 | 0.5 | 0.5 | 0.5 | 0.5 | 0.5 | 0.5 |
| | Vulcanization accelerator G*27 | 1 | 1 | 1 | 1 | 1 | 1 | 1 |
| | Sulfur*10 | 0.5 | 0.5 | 0.5 | 0.5 | 0.5 | 0.5 | 0.5 |
| | Foaming agent E*31 | 6 | 6 | 6 | 6 | 6 | 6 | 6 |
| | Foaming aid*12 | - | - | - | - | **3** | **3** | **3** |
| Expansion ratio index | | 100 | **103** | **109** | **110** | 100 | **118** | **120** |
| Average cell diameter index | | 100 | **51** | **58** | **61** | 100 | **88** | **82** |
| Tensile strength index | | 100 | **139** | **181** | **197** | 100 | **114** | **108** |

The details of each component in Table 5 are as described below.

*30: oil; Diana Process Oil PW-90, produced by Idemitsu Showa Shell

*31: component (b): foaming agent E; Unifoam AZ ULTRA #3170N-I (azodicarbonamide, median diameter: 15±4 um, surface-treated product), produced by Otsuka Chemical Co., Ltd.

Industrial Applicability

The rubber composition for producing a foam having a high expansion ratio of the present invention comprises a rubber component, a chemical foaming agent, a compound represented by formula (1) or a salt thereof, and at least one member selected from the group consisting of vulcanizing agents and crosslinking agents, whereby a high-expansion-ratio foam can be provided.

## Claims

1. A rubber composition for producing a foam having a high expansion ratio, comprising the following components (a), (b), (c), and (d) :

   (a) a rubber component;
   (b) a chemical foaming agent;
   (c) a compound represented by the following formula (1) or a salt thereof; and
   (d) at least one member selected from the group consisting of vulcanizing agents and crosslinking agents:

   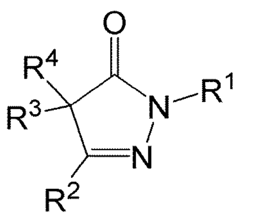
   (1)

   wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

2. The composition according to claim 1, wherein the rubber component is at least one diene rubber selected from the group consisting of natural rubber, styrene-butadiene copolymer rubber (SBR), butadiene rubber (BR), isoprene rubber (IR), styrene-isoprene-butadiene rubber (SIBR), nitrile rubber (NBR), chloroprene rubber (CR), a styrene-isoprene-styrene triblock copolymer (SIS), and a styrene-butadiene-styrene triblock copolymer (SBS).

3. The composition according to claim 1, wherein the rubber component is at least one non-diene rubber selected from the group consisting of butyl rubber, ethylene-propylene rubber (EPM), ethylene-propylene-diene terpolymer rubber (EPDM), urethane rubber (U), a propylene hexafluoride-vinylidene fluoride copolymer (FKM), a tetrafluoroethylene-propylene copolymer (FEPM), a tetrafluoroethylene-perfluorovinyl ether copolymer (FFKM), methyl silicone rubber (MQ), vinyl methyl silicone rubber (VMQ), phenyl methyl silicone rubber (PMQ), acrylic rubber (ACM), polysulfide rubber (T), and epichlorohydrin rubber (CO, ECO).

4. The composition according to any one of claims 1 to 3, wherein $R^1$, $R^3$, and $R^4$ are hydrogen atoms, and $R^2$ is a methyl group.

5. The composition according to any one of claims 1 to 4, further comprising component (e): a foaming aid.

6. A high-expansion-ratio foam foamed from the composition according to any one of claims 1 to 5.

7. A tire, an acoustic member, a sealing material, a hose, a belt, a wire covering, or a thermal insulation material, all of which are produced by using the composition according to any one of claims 1 to 5 or the foam according to claim 6.

8. An expansion ratio improver for rubber, comprising a compound represented by the following formula (1) or a salt thereof:

   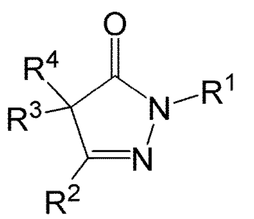
   (1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

**9.** A method for improving the expansion ratio of a foam, comprising mixing a rubber component, a chemical foaming agent, and a compound represented by the following formula (1) or a salt thereof:

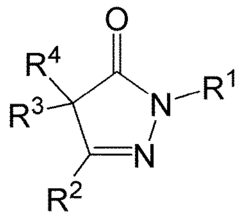

(1)

wherein $R^1$ represents a hydrogen atom, an alkyl group, or an aralkyl group; $R^2$, $R^3$, and $R^4$ are the same or different and each represents a hydrogen atom, an alkyl group, an aralkyl group, or an aryl group; and each of these groups optionally further has one or more substituents.

**10.** A method for producing a high-expansion-ratio foam, comprising:

step (A) of mixing a raw material component containing components (a) and (c); and
step (B) of mixing a mixture obtained in step (A) with components (b) and (d).

**11.** The method for producing a high-expansion-ratio foam according to claim 10, wherein the foam has an expansion ratio index of 103 or more.

**INTERNATIONAL SEARCH REPORT**

International application No.
**PCT/JP2021/047785**

**A. CLASSIFICATION OF SUBJECT MATTER**

***C08K 5/3445***(2006.01)i; ***C08L 21/00***(2006.01)i; ***C08J 9/04***(2006.01)i; ***C08J 9/06***(2006.01)i; ***C08K 3/011***(2018.01)i
FI: C08J9/06 CEQ; C08K5/3445; C08K3/011; C08J9/04 CEZ; C08L21/00; C08J9/04 103

According to International Patent Classification (IPC) or to both national classification and IPC

**B. FIELDS SEARCHED**

Minimum documentation searched (classification system followed by classification symbols)

C08K5/3445; C08L21/00; C08J9/04; C08J9/06; C08K3/011

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/045575 A1 (OTSUKA CHEMICAL CO LTD) 05 March 2020 (2020-03-05) examples | 8 |
| A | entire text | 1-7, 9-11 |
| X | JP 2008-189911 A (BRIDGESTONE CORP) 21 August 2008 (2008-08-21) examples | 8 |
| A | entire text | 1-7, 9-11 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 February 2022** | **15 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/047785**

| Patent document cited in search report | Publication date (day/month/year) | Patent family member(s) | Publication date (day/month/year) |
|---|---|---|---|
| WO 2020/045575 A1 | 05 March 2020 | CN 112533991 A<br>examples | |
| JP 2008-189911 A | 21 August 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020122160 A
- JP 2002165294 A